# EUROPEAN PATENT APPLICATION

(11) **EP 1 210 944 A1**
(43) Date of publication of application: **05.06.2002**
(21) Application number: 99974264.6
(22) Date of filing: 12.08.1999
(51) Int. Cl.: A61K 31/711, A61K 35/14, A61K 35/16, A61K 35/60

(54) **BLOOD PLASMA REPLACEMENT SOLUTION**

(71) Applicant: Asafov, Alexandr Vilenovich, Moscow, 117261 (RU); Asafova, Tatyana Dmitrievna, Moscow, 111261 (RU)
(72) Inventor: Asafov, Alexandr Vilenovich, Moscow, 117261 (RU); Asafova, Tatyana Dmitrievna, Moscow, 111261 (RU)
(74) Representative: Andrae, Steffen, Dr.
(86) International application number: RU9900289
(87) International publication number: WO0112201

(57) **Abstract**

PLASMA SUBSTITUTE COMPOSITION

The invention relates to medicine, more specifically the invention relates to pharmaceutical preparations usable for the treatment of severe acute and chronic lesions of a human body, which are the cause of hypovolemia or the alteration of functional properties of a blood with pronounced signs of the immunodeficiency.

The invention provides a plasma substitute composition having an immunomodulatmg effect. The invention provides the use of a specific biologically active ingredient agent which is a physiologically acceptable salt of a DNA derivative, preferably a sodium salt obtained from animal raw materials such as a sturgeon or salmon milt.

The proposed composition usable in an adjuvant therapy stimulates hematopoiesis and myelopoiesis without any side-effects or complications.

## Description

### Field of the invention

This invention relates generally to medicine, in particular to pharmaceutical preparations usable for the treatment of severe acute and chronic lesions of human body which are the cause of an extensive loss of blood and the alteration of its functional properties. More specifically, the invention relates to plasma substitute compositions.

### Background of the Invention

It is well known that severe acute lesions, such as extensive deep burns, necrotic tissue-induced sepsis caused by grave traumas, frosbites, penetrating wounds and the like, severe chronic lesions, such as various types of anemia, leucocytopenia, thrombocytopenia caused by a specific cancer-related chemotherapy in patients or by the action of radiation or poisoning during radial or ecological accidents, are normally associated with abnormal function of hemopoietic system and immunodeficiency.

A persistent immunodeficiency is also observed in patients with acute and chronic infectious diseases, such as AIDS, hepatitis B, genital herpes II and the like.

Besides adjunctive therapy of lesion focuses plasma substitute transfusion is the key mode of the treatment of such diseases.

Modern plasma substitute compositions may be arbitrary divided to four main groups based on their purpose and functional properties: solutions with a circulatory or antishock effect, desintoxication solutions, protein solution for parenteral nutrition and adjustors of water-salt/acid-base balance (see Mashkovsky M.D., "Medicinal Remedies": part 2, 122-135(1983); Manual for Transfusion of Blood and Blood Substitutes, Medicine, Moscow, p. 155-178, (1982)).

Among plasma substitutes with a circulatory effect a commonly used preparation is polyglucin which is a nontoxic/nonpyrogenic average-polymeric fraction of partially hydrolyzed dextran which has been dissolved in isotonic solution of sodium chloride.

Plasma substitute desintoxication compositions include, for example, hemodez or polydez which are solutions of a low molecular weight compound of polyvinylpyrolidone or polyvinyl alcohol, dissolved in suitable saline solution.
The most commonly used protein solutions for parenteral nutrition includes casein hydrolysate, hydrolysin, aminokrovin, each is a mixture of amino acids and simple peptides, dissolved in isotonic solution of sodium chloride and glucose.

Presently known adjustors of a water-salt and acid-base balance are solutions of Disol, Trisol, including a water-salt infusion solution of Lactasol which comprises the balanced saline and lactic acid.

Plasma substitute solutions have been also known to use for modeling the respiratory blood function (blood gas-transporting agents), which are prepared based on the concentrated solution of stroma/procoagulants-free hemoglobin (see Mashkovsky M.D., "Medicinal Remedies": part 2, 122-135(1983)). It has been developed in the art the modified hemoglobin-based model preparations for oxygen transport ( see US Patent 5,449,759, 09.12.1995), among which the emulsions of Perftoran and Perfucol are of special interest (Vesnik A.N. SSSR: v.6, p.55-64(1989)). Presently it has been developed a new starch-based plasma substitute solution with circulatory effect (see EP No. 059,633, 04.27.1994; US Patent No.5,470,841, 11.25.1995).

Due to the fact that various medical manipulations generally associated with extensive blood loss and grave shock should be performed simultaneously for filling the bloodstream, returning arterial pressure to normal and maintaining it at a stable level, eliminating toxins from the organism, normalizing the hemostasis, providing the transport of nutrients and oxygen, it gave rise to the development of a number of multimodal substitutes in the form of a solution, such as Polyfer with a circulatory and hemopoietic effects, or Rheogluman having diuretic and rheological effects.

Furthermore, in such cases the plasma substitute compositions are of a wide use to which there have been simultaneously added Polyglucin which raises the arterial tension and maintains the tension at a stable level, Rheopolyglucin which normalizes the microcirculation, eliminates the hemostasis and causes the redeposition of erythrocytes from capillars; compounds that helps to eliminate the exchange metabolites from the organism together with urine, and Lactasol, providing the elimination of tissue acidosis and the improvement of total therapeutic effect.
However, none of the prior art plasma substitute compositions, including the multifunctional plasma substitutes, could provide simultaneously the stimulation of the hemopoietic system and the restoration of functioning the immune system.

### Disclosure of the Invention

The invention is based on the problem of providing a new plasma substitute composition which, besides high regulatory properties, ensures the restoration and activation of hemopoietic system with showing the hemocorrecting and immunomodulating effects and capacity to normalize functioning the immune system. This permits one to increase in survival percentage, cut the time for treatment of patients and restore quickly the amount or functional properties of a blood.

This problem is solved by providing a plasma substitute composition comprising isotonic solution, which further contains a active ingredient having a hemocorrecting and immunomodulating effect and the capacity to activate hemopoiesis. As such active ingredient the composition comprises a physiologically acceptable salt of DNA derivative which is prepared from animal raw material.

To obtain the maximum efficiency the composition of the invention comprises sodium salt of the DNA derivative which is prepared from sturgeon or salmon milt.

This problem is further solved by providing a plasma substitute composition which comprises a physiologically acceptable salt of the DNA derivative having an average molecular weight (1.5-550)x10³ D and hyperchromic effect (28-47) %, with the composition contains the salt in the amount of (5x10³-5) g per 1 L of isotonic solution. According to the invention, the composition of the invention comprises a physiologically acceptable salt of the DNA derivative in an isotonic solution at the presence of a saline background.

This problem is further solved by providing a process for the preparation of a plasma substitute composition by preparing an isotonic solution to which is added an active ingredient, characterized in that a physiologically acceptable salt of the DNA derivative prepared from an animal raw material is further added to the isotonic solution in the amount and under conditions ensuring the efficiency of the solution, while maintaining its isotonic capacity, with a physiologically acceptable salt of the DNA derivative being preferably sodium salt.

For the improvement of the quality of the composition obtained, a sodium salt of the DNA derivative is pre-dissolved to the concentration which makes further dissolving of the resulting solution to be effective in the isotonic solution, with the content of the salt being (5x10⁻³-5) g per 1L of the solution and the content in the solution of:
protein - no more than 1.5% by weight, polysaccharides - no more than 2 % by weight, RNA- no more than 6 % by weight. A dissolvent includes distilled water or physiological solution.

The efficiency of the process may be increased, if a sodium salt of the DNA derivative having an average molecular weight (1.5-550)x10³ D and hyperchromic effect (28-47) % is added to an isotonic solution.

The claimed composition is a wide-spectrum plasma substitute, since almost all of the prior art plasma substitutes may be used in the form of isotonic solution. Moreover, by adding to the composition of the invention a novel active ingredient having both a hemocorrecting and immunomodulating activity allows such new preparations under use to show a surprising new effect which is characterized not only by promoting sharply the return to normal the blood count, and also by activating the function of hemopoietic system in considerably short time, with the subsequent effective immunodeficiency restoration, which would appear necessary at conditions associated with the risk for life.

### The embodiment of the invention

The composition of the present invention is prepared under factory conditions by the conventional volume-weight method.

According to the invention, the appropriate weight amount of a physiologically acceptable salt of the DNA derivative, for example sodium salt, with an average molecular weight (1.5-550)x10³ D and hyperchromic effect (28-47) %, obtained as a powder in which the content of protein is no more than 1.5% by weight, polysaccharides - no more than 2 % by weight, RNA- no more than 6 % by weight, is dissolved to the appropriate volume of the solution whose concentration is more than 0.5 % by weight. As the solvent there is used distilled water or physiological solution. The resulting solution is, then, filtered, sterilized and the appropriate amount of plasma substitute is added thereto until it reaches the appropriate concentration to provide the isotonic capacity of the solution.

The findings of the studies has revealed that the efficiency of the claimed solution depends on the quantitative content of the active ingredient , for example a sodium salt of the DNA derivative, which is added to the plasma substitute of the invention and its physicochemical characteristics as well (Table land 2), with the efficacy of it increases considerably, when a sodium salt of the DNA derivative having an average molecular weight (1.5-550)x10³ D and hyperchromic effect (28-47) % is added to an isotonic solution.

The so-obtained solution is a transparent colorless liquid, contains no mechanical contaminations or other impurities and is stable. It can be endured to long-term storage, and not less than 2 years. No contaminations have been observed under the chemical and bacteriological control.

**Table 1**

| Therapeutic effect as a function of quantitative content of the claimed active ingredient in the plasma substitute composition | | | |
|---|---|---|---|
| Content of the active ingredient in plasma substitute,% | > 0.5x 10⁻⁵ | 0.5x10⁻⁵- 0.5 | < 0.5 |
| Efficacy | Sharp decrease in therapeutic effect | Optimal therapeutic effect, no complications | Complications are possible, anaphylactic shock |

**Table 2**

| Therapeutic effect as a function of physicochemical characteristics of the added active ingredient | | | |
|---|---|---|---|
| Average molecular weight, D | > 1.5x10³ | 1.5x10³ -0 550x10³ | < 550x10³ |
| Efficacy | Decrease in therapeutic effects, increase in toxicity | Optimal therapeutic effect, no complications | Thrombocyte coagulation or aggregation is possible |

The experimental clinical studies revealed hemocorrecting effect of the claimed composition for the treatment of cytopenia induced by a cytostatic agent (a model of immunodeficiency caused by chronic environmental poisoning) or gamma-radiation (a model of immunodeficiency caused by radiological contamination).

The studies were carried out on male mice of F1 (CCBA x C₅₇B1) line, male rats F1 (AMCY x Wistar) and beagle hounds.

Cyclophosphan (home produced), Pharmorubicin (Pharm, Italia), Cisplatin (DDR, United States) were used as cytostatic agents. Irradiation was provided by a "Stebel-3a" apparatus. Cytostatic agents were administered intraperitoneally. During gamma-irradiation with "Stebel-3a" apparatus, 3-4 mice were placed into a glass chamber and introduced into irradiation zone of the apparatus with radiation sources arranged in the form of "squirrel cage". Dosimetric control showed that dose difference within the container was less than 10%. The irradiation dose was 6 Gy. The claimed composition was administered intravenously in a dose of 2 mg per 1 kg of animal weight.

Treatment by the claimed composition resulted in hemocorrecting and immunomodulating effects with clearly pronounced functional activation of hemopoietic system.

For example, morphological analysis of peripheral blood after administration of cytostatic agents showed that the claimed composition stimulated release lymphocytes and granulocytes into peripheral blood. On the day 6, the number of granulocutes in mice treated with the claimed composition was three times higher than in the control group (Table 3).

Effects of the proposed composition on dynamics of normalization of Blood Count after the total gamma-radiation are shown in Table 4. It is observable the statistically significant excess in leukocyte number in peripheral circulatory system, which is several time higher as compared with the Control group.

**Table 3**

| Effects of the claimed plasma substitute composition (C) with the active ingredient of the invention (K) on dynamics of changing lymphocytes and granulocyte count in peripheral circulatory system in mice in the presence of cyclophosphan (CP) | | | | | | |
|---|---|---|---|---|---|---|
| Dosage and regimen of using the preparations | Background Day 3 | Day 4 | Day 6 | Day 10 | Day 14 | Day 21 |
| | | Granulocyte | (%/ abs.c.) | | | |
| CP200mg/kg | 23±7.7 | 3.4±2.75 | 52.2±4.9 | 35±8.8 | 42.4±3.85 | 27.1±3.4 |
| CP200 mg/kg+ C&K 2 mg/kg | 2690±990 25±3.4 2875±390 | 56±45 4.2±2. 2 108±57 | 3500±330 64±8.2 10240±1310 | 2970±750 31.6±6 2780±530 | 4280±390 38.6±7.15 3980±740 | 3200±400 29.8±2.8 370±350 |

| | | Lymphocyte | (%/abs.c) | | | |
|---|---|---|---|---|---|---|
| CP200 mg/kg | 69.2±8.25 | 83.6±4.4 | 26.4±3.3 | 42±14.8 | 39.4±5.5 | 47.4±2.9 |
| CP200 mg/kg C&K | 8930±J070 71.3±5.8 | 1371±72 74±6 | 1770±220 20.4±3.85 | 3560±1250 48.6±6.6 | 3980±560 48.4±8.8 | 5600±340 66.1±5.2 |
| 2 mg/kg | 8199±667 | 1910±160 | 3260±620 | 4280±580 | 4980±906 | 8200±650 |

**Table 4**

| Effects of the claimed plasma substitute composition (C) on dynamics of changing leukocyte count in periferal bloodstream in mice under the total gamma-radiation at 600 Rad | | | | | | |
|---|---|---|---|---|---|---|
| Dosage and treatment regimen | Background Day 3 | Day 2 | Day 4 | Day 9 | Day 14 | Day 22 |
| | | Total | leukocyte | count | (th/mm³) | |
| Control group exposed to 600 Rad | 14.7±1.54 | 4.32±0.77 | 1.26±0.22 | 1.80±D.16 | 1.70±0.38 | 6.16±2.09 |
| Exposed to | 17.9±1.7 | 4.94±1.65 | 1.88±0.38 | 2.82±0.16 | 4.66±0.77 | 10.1±1.98 |
| 600 Rad +C & K | | | | | | |

Pharmacokinetic studies were carried out in mice, for which the conditions for production and purification of the active ingredient introduced into the composition in the form of physiologically acceptable salt of DNA derivative, mainly, tritium labeled sodium salt, were developed. Several pathways for administration of the pharmacological preparation, including intravenous, were used.

The concentration and time dependencies of the distribution of the labeled preparation its metabolites in blood, plasma, formed elements of blood, kidneys, liver, lymph nodes, spleen, thymus, stomach, small intestine, brain, bone, and bone marrow were studied. All pharmacokinetic curves, which describe changes in the concentration of this active ingredient in the form of physiologically acceptable salt of DNA derivative, mainly of sodium salt and its metabolites, in the studied organs and tissues, displayed a typical rapid phase of concentration decrease for 3-6 days. The highest tropism to the introduced active ingredient was found for the lymph nodes, spleen, stomach, and kidneys. An average retention time for the organs and tissues was equal to 60+2.9 hours. The active ingredient was released mainly with urine. Major amount of the active ingredient administered was excreted within the first day after the administration of the preparation: 37% and 3.3% of the dose with urine and feces, respectively. Further, the rate of the excretion process decreased, and by the sixth day after the administration of the preparation, animal body contained less than 10-15% of the administered dose. The administered active ingredient was completely eliminated from organs and tissues within, at least, ten days.

Toxicological properties of the claimed composition were extensively studied, with the acute toxicity region being studied in mice, rats, and dogs, the subacute toxicity studied in rats and dogs, the allergenic and immunological effects studied in guinea pigs, the embryotoxic and teratogenic effects studied in rats, pyrogenic properties studied in rabbits, as well as safety of the composition and potential mutagenic activity were studied with prognosis of carcinogenic activity.

The data obtained allow us to conclude that the claimed composition comprising a active ingredient having hemocorrecting and immunomodulating effects exhibit a low toxicity. Postmortem studies showed that changes in organs and tissues correlated with the dose to bbe used and were reversible. The claimed composition exerted no teratogenic, carcinogenic, or embryotoxic effect, did not affect the morphological and biochemical composition of peripheral blood, exhibited no gastrointestinal, cardio-, hepato-, and nephrotoxocity, did not exert local irritant action, and had no pyrogenic activity. Toxicity of the composition manifested itself only on its intravenous administration in inadmissible high doses and concentrations. The composition is not anaphylactogenic and does not induce sensitization on repeated administration.

These studies and their analysis allow the following major conclusion to be made:
- The use of the claimed composition induced activation of hemopoietic system on the background of hemocorrecting effect;
- unlike the known plasma substitute preparations, the claimed composition drastically accelerates recovery of the immune status of the body;
- no adverse reactions or complications were found on the application of the claimed composition. Clinical studies of the claimed composition were also carried out.

Clinical studies of the claimed invention were carried in patients suffering from chronic anemia and in oncological patients with hemopoiesis suppressed after chemio- and radiotherapy.

For clinical studies, patients suffering of chronic anemia were recruited at an age of above 18 years with anemia (males with erythrocyte number up to 4.0x10¹²/l; females with erythrocyte number up to 3.5x10¹²/l) at the time of inclusion, that or persisted for at least six months, or regularly recurred after the treatment within one year. Before the study, all patients passed complete medical examination with clinical hematological and biochemical tests to confirm the diagnosis: medical history; leukocyte, erythrocyte, and reticulocyte numbers; hemoglobin, hematocrit, complete blood count, ESR, iron and iron binding capacity of the serum, transferrin, ferritin, copper, zinc, and ceruloplasmin. Also, clinical immunological indices were determined that characterize the condition of the immune system: CD3, CD4, CD8, CD16, CD72-lumphocytes, serum immunoglobulines, phagocyte number and index, hemolytic activity of the complement, and titer of the rheumatoid factor.

Patients were treated by daily intravenous administration of 100-200 1 of polyglucin with the content in an isotonic solution of the active ingredient in the form of sodium salt of DNA derivative in an amount of (0.5-1.5)x10⁻³, for three days.

After treatment, the results was assessed by the following criteria: health condition and subjective feeling of patient, dynamics of the hematological characteristics, dynamics of the immunological characteristics, dynamics of the biochemical characteristics, recurrence of the disease within the follow-up period.

After summarization of the data for 18 patients, statistically significant positive dynamics with subsequent normalization of hematological, biochemical and immunological characteristics in 83% of the patients with signs of the functional activation of hemopoietic system, other patients showed positive dynamics of these characteristics with non-pronounced relapse of the disease 40-60 days after termination of the course of treatment. A hyperthermic response, with temperature increase to 38-38.5°, was found in 22% of the patients; the response spontaneously disappeared within 2-3 days without administration of symptomatic treatment. No other adverse effects were observed.

For studies in oncological patients, a group of 14 patients was selected, who received combined chemotherapy in the intense CAM regime against small cell lung carcinoma. Before beginning the study, the patients passed complete clinical examination: lung roentgenography, CT of the liver, adrenals, brain, bone scanning, biochemical blood analysis, and myelogram.

On the first day of treatment, patients with morphologically documented localized small cell lung carcinoma received the following preparations: Cyclophosphanum 1.5 g/m² i/v, Adriamycin 60 mg/m² i/v, Methotrexate 30 mg/m² i/v.

The course of treatment was performed by daily intravenous administration of 100 - 200 ml of Rheopolyglucin with the content of the administered active ingredient (0.5 - 1.5)x10⁻³%, starting from the second day of treatment for 5 - 10 days. Blood counts were made daily from the 2^{nd} to 20th day, on the days 6 and 10, control mielogram was recorded. During the study, the patients received no other hemostimulating preparations, vitamins, corticosteroids, and blood transfusion.

The total data showed that administration of the claimed preparation in patients with small cell lung carcinoma on the background of chemotherapy resulted in a lesser intensity and a shorter duration of leuko- and neutropenia, avoid septic complications during the chemotherapy, and, allowed repeated courses of chemotherapy to be started in 64% of the patients with an interval between the courses shorter by 20 - 30%. All patients reported satisfactory tolerability of the preparation, temperature rise to 38° was recorded in 21% patients, which disappeared spontaneously, did not require symptomatic treatment and did not prevent further administration of the preparation.

Specific embodiment of use of the present invention in patients is illustrated by the following example.

### Example

Patient K.A.y., 1935 year of birth, chronic anemia, under observation - 6 months, the efficacy after the administration of the commonly used preparations is short or absent. Received the treatment with the plasma substitute composition by daily intravenous injection of 200 ml of Polyglucin with the content of the administered active ingredient 0.5x10⁻³ % during the course of 3 days. The results are shown in Table 5.

**Table 5**

| | Background | Day 9 | Day 13 | Day 20 | Day 50 |
|---|---|---|---|---|---|
| Erythrocytes | 3.05 | 3.34 | 3.48 | 3.75 | 4.06 |
| ESR | 19 | 52 | 44 | 24 | 19 |
| Leukocytes | 3400 | 4400 | 4400 | 4700 | 5100 |
| Stab % | 4 | 6 | 2 | 4 | 5 |
| Segm. % | 53 | 59.5 | 62 | 70 | 46 |
| Eosiophils % | 4 | 1 | 1 | 1 | 2 |
| Basophils % | 0 | 0.5 | 0 | 0 | 0 |
| Monocytes % | 5 | 6.5 | 3 | 6 | 11 |
| Lymphocytes% | 34 | 26.5 | 33 | 19 | 36 |
| Segm.abs. | 1938 | 2882 | 2816 | 3478 | 1394 |
| Eosiophils abs | 136 | 44 | 44 | 47 | 55 |
| Basophils abs | 0 | 22 | 0 | 0 | 0 |
| Monocytes abs | 170 | 286 | 132 | 282 | 301 |
| Lymphocytes abs | 1156 | 1166 | 1452 | 893 | 984 |
| CD3 % | 33 | | | 40.5 | |
| CD4% | 8 | | | 21.6 | |
| CD8% | 29 | | | 41.2 | |
| CD72 % | 17 | | | 33.3 | |
| CD16 % | 31 | | | 24 | |
| CD3 abs | 381 | | | 362 | |
| CD4 abs | 92 | | | 193 | |
| CD8 abs | 335 | | | 338 | |
| CD72abs | 197 | | | 297 | |
| CD 16abs | 358 | | | 214 | |
| Phage number | 90 | | | 96 | |
| Phage index | 6.1 | | | 12 | |

The above example is characterized by a persistent increase in the erythrocyte and leukocyte numbers for 50 days of the follow-up, and normalization of other indices. Noted was the permanent improvement in the state of health of the patient, as well as disappearance of dizziness and weakness symptoms. No general complications or adverse effects was noted. These data allow us to recommend this preparation for wide use as the plasma substitute, having hemocorrecting and immunomodulating effects .

Studies in other patients confirmed the efficacy of the claimed composition for activation of hemopoietic system and recovery of the immune state of patients, thereby attaining the object of the invention.

### Industrial applicability of the invention

The plasma substitute composition can find a wide use in medicine, veterinary for the treatment of severe acute and chronic lesions of the body, which results to hypovolemia and alteration of functional properties of the blood followed by the signs of the immunodeficiency.

## Claims

1. Plasma substitute composition, comprising an isotonic solution, **characterized in that** it further comprises an active ingredient having hemocorrecting and immunomodulating effects, and which is capable to activate hemopoietic system.

2. Plasma substitute composition of Claim 1, **characterized in that** said active ingredient is a physiologically acceptable salt of the DNA derivative which is prepared from an animal raw material.

3. Plasma substitute composition of Claim 2, **characterized in that** it comprises sodium salt of the DNA derivative.

4. Plasma substitute composition of any Claims 1, 2 or 3, **characterized in that** it comprises a physiologically acceptable salt of the DNA derivative, which is prepared from a sturgeon milt.

5. Plasma substitute composition of any Claims 1, 2 or 3, **characterized in that** it comprises a physiologically acceptable salt of the DNA derivative, which is prepared from a salmon milt.

6. Plasma substitute composition of any Claims 1, 2 or 3, **characterized in that** it comprises a physiologically acceptable salt of the DNA derivative, which is prepared from a sturgeon or salmon milt.

7. Plasma substitute composition of any Claims 1 or 2, **characterized in that** it comprises a physiologically acceptable salt of the DNA derivative having an average molecular weight (1.5-550)x10³ D and hyperchromic effect (28-47) %.

8. Plasma substitute composition any Claims 2 or 6, **characterized in that** it comprises a physiologically acceptable salt of the DNA derivative 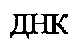 in the amount of (5x10³-5) g per 1 L of isotonic solution.

9. Plasma substitute composition of any claims 2 or 7, **characterized in that** it comprises a physiologically acceptable salt of the DNA derivative in isotonic solution in the presence of a salt background.

10. A process for the preparation of plasma substitute composition of Claim 1 by preparing an isotonic solution to which an active ingredient is added, **characterized in that** it comprises also the addition to the resulting isotonic solution a physiologically acceptable salt of the DNA derivative prepared from an animal raw material in an amount and under conditions ensuring the efficiency of the solution, while maintaining its isotonic capacity.

11. A process for the preparation of plasma substitute composition of Claim 9, **characterized in that** the additionally added physiologically acceptable salt of the DNA derivative is a sodium salt of the DNA derivative.

12. A process of Claim 10, **characterized in that** a sodium salt of the DNA derivative having an average molecular weight (1.5-550)x10³ D and hyperchromic effect (28-47) % is added to the isotonic solution.

13. A process of Claim 10, **characterized in that** the physiologically acceptable salt of the DNA derivative is pre-dissolved to the concentration which makes further dissolving of the resulting solution to be effective in the isotonic solution, with the content of the salt being (5x10⁻³-5) g per 1L of the solution and the content in the resulting solution of:
protein is no more than 1.5% by weight, polysaccharides is no more than 2 % by weight, RNA- no more than 6 % by weight.
